# FASCICULE DE BREVET EUROPEEN

(11) **EP 1 392 220 B1**
(45) Date de publication et mention de la délivrance du brevet: **27.02.2008**
(21) Numéro de dépôt: 01934080.1
(22) Date de dépôt: 10.05.2001
(51) Int. Cl.: A61K 8/11, A61K 8/97, A61Q 19/08

(54) **PRODUIT COSMETIQUE, NOTAMMENT POUR LES SOINS DE LA PEAU**
KOSMETISCHES MITTEL, INSBESONDERE FÜR DIE HAUTPFLEGE
COSMETIC PRODUCT, IN PARTICULAR FOR SKIN CARE

(30) Priorité: 10.05.2000 FR 0005939
(43) Date de publication de la demande: 03.03.2004
(73) Titulaire: Fauvel, Michel, 72000 Le Mans (FR); Drouet, Marcelle, 72210 Fille s/Sarthe (FR)
(72) Inventeur: Fauvel, Michel, 72000 Le Mans (FR); Drouet, Marcelle, 72210 Fille s/Sarthe (FR)
(86) Numéro de dépôt international: PCT/FR2001/001416
(87) Numéro de publication internationale: WO 2001/085101

(56) Documents cités:
- EP-A- 0 085 589
- DE-A- 19 756 499
- FR-A- 2 744 915
- US-A- 4 798 786
- US-A- 5 484 721

## Description

L'invention se rapporte aux produits d'hygiène et de beauté.

Elle concerne plus particulièrement un produit cosmétique, notamment pour les soins de la peau, propre notamment à empêcher le vieillissement de la peau et à protéger la peau contre les agressions extérieures.

On connaît déjà des produits cosmétiques de ce genre qui se présentent le plus souvent sous la forme d'une crème, d'un lait, d'une lotion, d'un gel, d'une huile, etc. Ces produits contiennent un ou plusieurs principes actifs en mélange avec un véhicule physiologiquement acceptable.

Ces produits cosmétiques connus comprennent généralement un ou plusieurs principes actifs d'origine naturelle et/ou synthétique incorporés dans une base, qui contient généralement une phase aqueuse et/ou une phase grasse, pour constituer un produit à appliquer sur la peau.

Dans les produits connus de ce type, les principes actifs sont habituellement simplement mélangés avec le véhicule, si bien qu'ils ont tendance à perdre une grande part de leur efficacité. En outre ces produits ne permettent pas de libérer progressivement les principes actifs.

Il en résulte que ces produits connus ne permettent pas de contrôler efficacement la libération dans le temps des principes actifs à appliquer sur la peau.

L'invention a notamment pour but de surmonter les inconvénients précités.

Elle vise en particulier à procurer un produit cosmétique qui permet de conserver l'efficacité totale du principe actif jusqu'au moment de son application.

L'invention vise également à procurer un produit cosmétique qui permet de libérer, dans des conditions contrôlées, le principe actif à appliquer sur la peau.

Elle vise aussi à procurer un produit cosmétique de ce type dans lequel le principe actif provient d'un produit naturel.

L'invention propose à cet effet un produit cosmétique, notamment pour les soins de la peau, lequel comprend, en tant que principe actif, des cellules végétales à teneur en polyphénols qui sont encapsulées dans des micro-particules dégradables aptes à libérer progressivement le principe actif lors de l'application dudit produit.

Ainsi, le produit cosmétique de l'invention tire partie des propriétés avantageuses des polyphénols que l'on retrouve dans certaines cellules végétales. Il permet, grâce à l'encapsulation des cellules végétales, de conserver l'efficacité totale du principe actif et de libérer progressivement ce dernier lorsque le produit est appliqué sur la peau.

Il en résulte un produit cosmétique dont le principe actif est d'origine naturelle et dont la libération peut s'effectuer dans des conditions contrôlées, ce qui permet de prolonger dans le temps l'efficacité de ce principe actif.

L'invention repose donc essentiellement sur la combinaison de cellules végétales à teneur en polyphénols et de micro-particules dégradables qui permettent d'assurer une libération progressive de ces cellules, et donc des principes actifs qu'elles contiennent, lorsque le produit est appliqué sur la peau.

Lorsque le produit est appliqué, les polyphénols qu'il contient sont alors en mesure d'exercer leurs propriétés avantageuses, notamment leurs propriétés anti-radicalaires et anti-oxydantes.

En raison de leur teneur en polyphénols, on préfère tout particulièrement utiliser des cellules végétales provenant de l'une au moins des plantes appartenant aux genres suivants :
Vitis sp. (vigne)
Quercus sp. (chêne)
Crataegus monogyna (aubépine)
Onobrychis victifolia (sainfoin)
Lotus corniculatus (lotier)
Ginkgo biloba (ginkgo)
Fagopyrum esculentum (sarrasin)
Cinnamonum cassia (cannelle)
Fragaria sp. (fraisier)
Pinus sp. (pin)
Picea sp. (epicéa)
Camelia sinensis (thé).

Dans la liste ci-dessus, le terme "sp.", qui veut dire "species", signifie que l'on ne précise pas l'espèce. Ainsi toutes les espèces du genre (qui est le premier nom) sont comprises.

Dans le cadre de l'invention, on préfère tout particulièrement, en raison de leurs propriétés avantageuses, les cellules végétales qui proviennent de plantes du genre Vitis (vigne), et en particulier de l'espèce Vitis vinifera.

Il est apparu en effet que les cellules de vigne, qu'il s'agisse de vignes cultivées, quel que soit le cépage, ou encore de vignes sauvages, quel que soit leur pays d'origine, ont une composante majeure en polyphénols.

Ces polyphénols se répartissent en sept grands groupes présents dans les cellules, à savoir : les catéchines, les épicatéchines, l'épicatéchine gallate, l'épigallocatéchine-SH, la catéchine-SH, et l'épicatéchine gallate-SH.

Ces cellules végétales ont pour avantage de disposer de principes actifs très performants, qui proviennent directement de la plante, sans nécessiter aucune extraction d'origine chimique par solvant. Il en résulte que, dans le produit cosmétique, ces cellules végétales sont directement disponibles.

Dans l'invention, les cellules végétales incorporées dans les micro-particules peuvent être soit des cellules vivantes (encore appelées cellules fraîches), soit des cellules congelées, soit encore des cellules lyophilisées.

Selon une autre caractéristique de l'invention, ces cellules végétales proviennent d'une culture in vitro et sont ajoutées aux micro-particules après filtration. La culture in vitro est menée dans des conditions contrôlées et dans un milieu de culture choisi pour favoriser la production des polyphénols.

Les cellules végétales, ainsi séparées de leur milieu de culture, sont incorporées directement en tant que telles aux micro-particules.

De la sorte, les principes actifs de ces cellules végétales sont contenus directement dans ces micro-particules, ce qui permet de conserver leur efficacité. Ils sont donc susceptibles d'agir sur la peau lorsqu'ils sont libérés du fait de la dégradation des micro-particules.

Selon une autre caractéristique de l'invention, les micro-particules comprennent une matrice particulaire biodégradable.

De préférence, cette matrice particulaire biodégradable est de type polysaccharidique.

Dans une forme de réalisation préférée, la matrice de type polysaccharidique comprend des maltodextrines à réticulation glycérique (glycéryl dimaltodextrine).

Le produit cosmétique selon l'invention peut être réalisé sous différentes formes convenant à une application topique.

A ce titre, on préfère tout particulièrement un produit cosmétique sous la forme d'une crème, ou encore sous la forme d'un lait. Mais il est possible aussi de réaliser d'autres produits cosmétiques, en particulier des lotions, des gels, des huiles, des produits de maquillage, etc.

Lorsque le produit cosmétique est appliqué sur la peau, les micro-particules sont dégradées par hydrolyse et/ou par action mécanique, ce qui libère progressivement les principes actifs, c'est à dire les polyphénols.

On décrira maintenant l'invention en référence aux exemples de réalisation suivants.

Ces exemples sont donnés seulement à titre illustratif et ne doivent en aucune façon limiter la portée de l'invention. Dans ces exemples, les pourcentages sont exprimés en poids sauf indication contraire.

### Exemple 1

### Préparation de cellules végétales (cellules de vigne)

Dans cet exemple, on part de souches de cellules de vigne (Vitis vinifera) appartenant à des cépages riches en polyphénols, obtenues à partir de pétioles ou de jeunes tiges prélevées en champ.

On suit le protocole général, tel qu'indiqué ci-dessous, pour la production de cultures végétales in-vitro.

Des suspensions cellulaires de vigne sont repiquées au laboratoire dans des fioles d'Erlenmeyer, dans des conditions stériles, chaque semaine dans un milieu de culture appelé "milieu d'entretien" permettant la survie et la croissance des cellules. Ce milieu de culture apporte des éléments minéraux, des vitamines, des phytohormones et du saccharose, suivant des quantités connues et exactement pesées au laboratoire.

Ces cellules en suspension se présentent sous la forme de petits agrégats de quelques cellules à quelques dizaines de cellules, la dimension d'une cellule étant d'environ 50 micromètres.

Pour permettre une culture en bio-réacteur, et obtenir des cellules accumulant des polyphénols, on utilise un milieu de culture stimulant la culture. Ce milieu, appelé "milieu de production", est identique au milieu d'entretien, sauf que sa teneur en saccharose est multipliée par trois et que ses teneurs en sulfate d'ammonium et phosphate de sodium sont multipliées par deux.

Les conditions de culture dans le bio-réacteur sont les suivantes :
- conditions de culture stériles,
- procédé du type en discontinu,
- aération de 0,1 à 0,2 vvm (où vvm = volume d'air/volume du milieu/minute),
- agitation de 70 à 100 tours/minute avec une turbine à pales inclinées ou une turbine classique à pales droites du type Rushton,
- rapport quantité d'inoculum/milieu de production = 1/5 à 1/8,
- durée de la culture : 14-18 jours,
- température = 25° C,
- volume du bio-réacteur : 2 litres à 400 litres.

Après la culture, les cellules végétales sont filtrées pour éliminer le milieu de culture résiduel et sont rincées à l'eau distillée froide. On obtient une biomasse d'environ 350 grammes de matière fraîche par litre de culture ou 25 grammes de matière sèche (après lyophilisation). La teneur en polyphénols est d'environ 0,4 à 8% par rapport au poids de la matière. Ces polyphénols sont stockés dans la vacuole des cellules de vigne.

### Préparation des micro-particules

On prépare des micro-particules conformément aux enseignements de la publication WO 98/13030 (exemple 13).

Tout d'abord, on prépare des matrices polysaccharidiques cationiques et réticulées de la façon décrite ci-après.

Dans un réacteur de 2,5 litres, on disperse 100 grammes d'amidon ayant un poids moléculaire d'environ 10.000 dans 300 millilitres d'eau contenant 1 gramme de borohydrure de sodium. Après deux heures d'agitation, on ajoute 100 millilitres d'une solution de soude 4N. Lorsque la solution est homogène, on ajoute 32 millilitres d'une solution aqueuse de chlorure de (2,3 époxypropyl) triméthylammonium à 75%. Après deux heures d'agitation, on ajoute 5 millilitres d'épichlorhydrine en maintenant l'agitation.

La solution est dispersée dans deux litres de dichlorométhane sous agitation suffisante pour obtenir une dispersion de la phase aqueuse sous la forme de gouttelettes de taille comprise entre 50 et 500 micromètres. La dispersion est maintenue sous agitation à température ambiante pendant 14 heures. La dispersion est alors filtrée et les matrices sphériques reprises dans deux litres d'éthanol 50% et le pH est ajusté à 5 par addition d'acide chlorhydrique 2N. Elles sont ensuite lavées deux fois dans 5 litres d'éthanol 20% puis deux fois dans 5 litres d'eau distillée à 50°C.

### Préparation des micro-particules incorporant les cellules végétales

Sous une hotte à flux laminaire, 50 grammes de cellules végétales, telles qu'obtenues précédemment, sont filtrés par simple gravitation (porosité 5 micromètres) puis ajoutées en fin de fabrication à 100 grammes de micro-particules (matrice polysaccharidique réticulée) préparées comme indiqué ci-dessus.

On obtient ainsi des micro-particules sous forme hydratée qui contiennent 85 à 95% d'eau liée au réseau constitutif, le reste de leur composition moyenne étant de 4 à 8% de polysaccharides modifiés et de 1 à 10% de principe actif (Vitis vinifera). Les micro-particules ainsi obtenues peuvent être soumises à une étape de déshydratation plus ou moins poussée, les micro-particules sèches étant stables et retrouvant leurs caractéristiques initiales après réhydratation.

Les micro-particules ainsi obtenues peuvent être incorporées dans des produits cosmétiques convenant essentiellement à une application topique sur la peau.

On préfère tout particulièrement des produits cosmétiques qui se présentent sous la forme d'une crème, d'un lait, d'une lotion, d'un gel, d'une huile, etc.

Les micro-particules sont incorporées à un véhicule approprié qui forme la base du produit cosmétique et qui, dans la plupart des cas, se compose principalement d'une phase aqueuse et/ou d'une phase grasse, d'un conservateur et d'autres additifs habituels, connus dans ce domaine.

La proportion de cellules végétales dans le produit cosmétique dépend de la nature de ce produit et de celle des soins à prodiguer. Elle est généralement comprise entre 3 et 10% en poids et peut atteindre des valeurs plus élévées, par exemple de 25%, pour des soins intensifs.

### Exemple 2

### Produit cosmétique du type crème

On prépare un produit cosmétique de type crème, qui comprend essentiellement les composants suivants :
- phase aqueuse A composée essentiellement d'eau désionisée,
- phase grasse B comprenant essentiellement un émulsionnant,
- phase C, comprenant essentiellement un conservateur, et
- phase D qui comprend essentiellement le produit actif sensible.

Dans l'exemple, cette phase D comprend essentiellement des micro-particules de cellules de vignes encapsulées et lyophilisées.

Elle peut aussi contenir d'autres principes actifs et/ou un parfum approprié.

### Exemple 3

### Produit cosmétique du type lait

On prépare un lait qui contient essentiellement les composants suivants :
- phase aqueuse A, essentiellement à base d'eau désionisée,
- phase grasse B, comprenant essentiellement un émulsionnant et un émollient,
- phase C, comprenant un conservateur et un produit hydratant, et
- phase D, comprenant essentiellement les produits actifs, à savoir des cellules de vigne encapsulées et lyophilisées.

Bien entendu, l'invention n'est pas limitée aux exemples de réalisation donnés précédemment à titre illustratif et s'étend à d'autres variantes.

Ainsi, l'invention peut s'appliquer à d'autres types de cellules végétales que des cellules de vigne, à condition que ces cellules végétales contiennent des polyphénols en quantité suffisante pour pouvoir exercer une action sur la peau.

Egalement, l'invention n'est pas limitée à des cellules végétales sous forme lyophilisée, et s'applique aussi à d'autres types de cellules végétales, du moment qu'elles sont encapsulées dans des micro-particules susceptibles de libérer progressivement le principe actif.

A ce propos, on a intérêt à utiliser des micro-particules qui sont de type biodégradable et libèrent le principe actif, soit par hydrolyse, soit encore par effet mécanique, par exemple par massage sur la peau.

Les produits cosmétiques de l'invention exercent une action bénéfique sur la peau, du fait des propriétés anti-radicalaires et anti-oxydantes des polyphénols qui sont contenus, en quantité plus ou moins importantes, dans les cellules végétales.

Ceci vaut spécialement pour les cellules de vigne qui trouvent un intérêt tout particulier dans le cadre de cette invention.

Egalement, il est possible d'associer, à ces cellules végétales,' d'autres principes actifs, qu'ils soient ou non d'origine végétale.

Il a été constaté que les produits cosmétiques de l'invention offrent des propriétés intéressantes qui permettent, de par leurs propriétés anti-radicalaires et anti-oxydantes, de retarder le vieillissement de la peau, d'hydrater ou d'apaiser la peau, de protéger la peau à l'égard des agressions extérieures, ou encore d'activer la régénérescence cellulaire de la peau.

Ces produits cosmétiques offrent également des propriétés nourrissantes, tonifiantes, neurotoniques et décontractantes vis à vis de la peau.

Dans tous les cas, il en résulte une amélioration des propriétés de la peau, cette amélioration étant prolongée dans le temps du fait de la libération progressive du principe actif par les micro-particules.

Le produit cosmétique de l'invention peut être réalisé aussi sous la forme d'un produit de maquillage (rouge à lèvres, poudre, fond de teint, etc) ou d'un produit capillaire.

## Revendications

1. Produit cosmétique, notamment pour les soins de la peau, **caractérisé en ce qu'**il comprend en tant que principe actif des cellules végétales à teneur en polyphénols qui sont encapsulées dans des micro-particules dégradables aptes à libérer progressivement le principe actif lors de l'application dudit produit sur la peau.

2. Produit cosmétique selon la revendication 1, **caractérisé en ce que** les cellules végétales proviennent de l'une au moins des plantes appartenant aux genres suivants :
Vitis sp. (vigne)
Quercus sp. (chêne)
Crataegus monogyna (aubépine)
Onobrychis victifolia (sainfoin)
Lotus corniculatus (lotier)
Ginkgo biloba (ginkgo)
Fagopyrum esculentum (sarrasin)
Cinnamonum cassia (cannelle)
Fragaria sp. (fraisier)
Pinus sp. (pin)
Picea sp. (epicéa)
Camelia sinensis (thé).

3. Produit cosmétique selon l'une des revendications 1 et 2, **caractérisé en ce que** les cellules végétales proviennent de plantes du genre Vitis (vigne), et en particulier de l'espèce Vitis vinifera.

4. Produit cosmétique selon l'une des revendications 1 à 3, **caractérisé en ce que** les cellules végétales sont des cellules vivantes (fraîches).

5. Produit cosmétique selon l'une des revendications 1 à 3, **caractérisé en ce que** les cellules végétales sont des cellules congelées.

6. Produit cosmétique selon l'une des revendications 1 à 3, **caractérisé en ce que** les cellules végétales sont des cellules lyophilisées.

7. Produit cosmétique selon l'une des revendications 1 à 6, **caractérisé en ce que** les cellules végétales proviennent d'une culture in vitro et sont ajoutées aux micro-particules après filtration.

8. Produit cosmétique selon l'une des revendications 1 à 7, **caractérisé en ce que** les micro-particules comprennent une matrice particulaire bio-dégradable, en particulier de type polysaccharidique.

9. Produit cosmétique selon la revendication 8, **caractérisé en ce que** la matrice de type polysaccharidique comprend des maltodextrines à réticulation glycérique (glycéryl dimaltodextrine).

10. Produit cosmétique selon l'une des revendications 1 à 9, **caractérisé en ce qu'**il est réalisé sous la forme d'une crème, d'un lait, d'une lotion, d'un gel, d'une huile ou d'un produit de maquillage.

## Claims

1. Cosmetic product, notably for skincare, characteristic in that it comprises as an active ingredient vegetable cells containing polyphenols that are encapsulated in degradable micro-particles, suitable for progressively releasing the active ingredient during the application of the aforementioned product on the skin.

2. Cosmetic product in accordance with claim 1, characteristic in that the vegetable cells come from at least one of the plants belonging to the following genera:
Vitis sp. (vine)
Quercus sp. (oak)
Crataegus monogyna (hawthorn)
Onobrychis victifolia (sainfoin)
Lotus corniculatus (lotus)
Ginko biloba (ginko)
Fagopyrum esculentum (buckwheat)
Cinnamonum cassia (cinnamon)
Fragaria sp. (strawberry plant)
Pinus sp. (pine)
Picea sp. (spruce)
Camelia sinensis (tea).

3. Cosmetic product in accordance with one of the claims 1 and 2, characteristic in that the vegetable cells come from plants of the genus Vitis (vine), and in particular from the species Vitis Vinifera.

4. Cosmetic product in accordance with one of the claims 1 to 3, characteristic in that the vegetable cells are living (fresh) cells.

5. Cosmetic product in accordance with one of the claims 1 to 3, characteristic in that the vegetable cells are frozen cells.

6. Cosmetic product in accordance with one of the claims 1 to 3, characteristic in that the vegetable cells are freeze-dried cells.

7. Cosmetic product in accordance with one of the claims 1 to 6, characteristic in that the vegetable cells come from an in vitro culture and are added to the micro-particles after filtration.

8. Cosmetic product in accordance with one of the claims 1 to 7, characteristic in that the micro-particles comprise a bio-degradable particle matrix, specifically of the polysaccharide type.

9. Cosmetic product in accordance with claim 8, characteristic in that the polysaccharide-type matrix comprises glyceric cross-linkage maltodextrins (glyceryl dimaltodextrin).

10. Cosmetic product in accordance with one of the claims 1 to 9, characteristic in that it is produced in the form of a cream, a milk, a lotion, a gel, an oil or a make-up product.

## Patentansprüche

1. Kosmetikprodukt, vor allem zur Hautpflege; **dadurch charakterisiert,** was es als Wirkstoff pflanzlicher, polyphenolhaltiger Zellen enthält, die in abbaubaren Mikro-Partikeln (die sich dazu eignen, den Wirkstoff schrittweise im Rahmen der Anwendung des besagten Produkts freizusetzen) eingekapselt sind

2. Kosmetikprodukt, gemäß Forderung 1, das durch die pflanzlichen Zellen sowie der Tatsache charakterisiert wird, dass sie wenigstens einer der folgenden Pflanzenarten angehören:
- Vitis sp. (Weinstock)
- Quercus sp. (Eiche)
- Crataegus monogyna (Hagedorn)
- Onobrychis victifolia (Esparsette)
- Lotus corniculatus (Hornklee)
- Gingko biloba (Gingko)
- Fagopyrum esculentum (Buchweizen)
- Cinnamonum cassia (Zimt)
- Fragaria sp. (Erdbeerstrauch)
- Pinus sp. (Pinie)
- Picea sp. (Rottanne)
- Camelia sinensis (Tee)

3. Kosmetikprodukt gemäß einer der Forderungen 1 oder 2, **dadurch charakterisiert, dass** die Frischzellen von Pflanzen des Typs Vitis (Weinstock) und vor allem aus dem Raum Vitis vinifera stammen.

4. Kosmetikprodukt gemäß einer der Forderungen 1 bis 3, **dadurch charakterisiert, dass** die Frischzellen Lebzellen (frisch) sind.

5. Kosmetikprodukt gemäß einer der Forderungen 1 bis 3, **dadurch charakterisiert, dass** die pflanzlichen Zellen gefrorene Zellen sind.

6. Kosmetikprodukt gemäß einer der Forderungen 1 bis 3, **dadurch charakterisiert, dass** die pflanzlichen Zellen gefriergetrocknete Zellen sind.

7. Kosmetikprodukt gemäß einer der Forderungen 1 bis 6, **dadurch charakterisiert, dass** die pflanzlichen Zellen aus einer in vitro-Kultur stammen und den Mikro-Partikeln nach Filterung zugefügt werden.

8. Kosmetikprodukt gemäß einer der Forderungen 1 bis 7, **dadurch charakterisiert, dass** die Mikro-Partikel teilweise eine biologisch abbaubare Matrix (vor allem des Typs Polysaccharid) beinhalten.

9. Kosmetikprodukt gemäß Forderung 8, **dadurch charakterisiert, dass** die Matrix des Typs Polysaccharid Maltodextrine mit glycerischer Anlagerung (Dimaltodextro-Glyceryl) beinhaltet.

10. Kosmetikprodukt gemäß einer der Forderungen 1 bis 9, **dadurch charakterisiert, dass** es in Form einer Creme, einer Milch, einer Lotion, eines Gels, eines Öls oder einer Schminkproduktes realisiert wird.
